# EUROPÄISCHE PATENTSCHRIFT

(11) **EP 2 028 171 B1**
(45) Veröffentlichungstag und Bekanntmachung des Hinweises auf die Patenterteilung: **05.05.2010**
(21) Anmeldenummer: 08104747.4
(22) Anmeldetag: 14.07.2008
(51) Int. Cl.: C07C 7/00, F25J 3/02

(54) **Verfahren und Vorrichtung zur Herstellung von flüssigem und/oder gasförmigem Methan**
Method and device for the manufacture of fluid and/or gaseous methane
Procédé et dispositif de fabrication de méthane liquide et/ou gazeux

(30) Priorität: 12.07.2007 DE 102007032536
(43) Veröffentlichungstag der Anmeldung: 25.02.2009
(73) Patentinhaber: Biogas Süd Entwicklungsgesellschaft OHG, 83233 Bernau (DE)
(72) Erfinder: Finsterwalder, Klemens, Dr., 83233, Bernau / Hittenkirchen (DE)
(74) Vertreter: Molnia, David

(56) Entgegenhaltungen:
- US-A- 5 956 971

## Beschreibung

Die Erfindung bezieht sich auf eine Vorrichtung und ein Verfahren zur Abtrennung von Methan aus methanhaltigen Gemischen, deren Komponenten eine Siede-, Schmelz- oder Sublimationstemperatur haben, die höher ist als die Siedetemperatur von flüssigem Methan, durch das Ausfrieren dieser Komponenten auf der Oberfläche von einem oder mehreren Wärmetauschern, in welchen tiefkalt verflüssigtes Methan (*liquefied natural gas,* LNG) als Kühlmittel verwendet wird. Insbesondere bei methanhaltigen Gasgemischen, deren weitere Komponenten überwiegend aus Kohlendioxid bestehen, kann mit der Vorrichtung und dem Verfahren gemäß der vorliegenden Erfindung außerdem Trockeneis gewonnen werden. Erfindungsgemäß kann weiterhin das abgetrennte Trockeneis als Kältequelle in einer Vorbehandlungseinheit zur Trocknung und Kühlung des methanhaltigen Gasgemisches verwendet werden.

Gasgemische, welche zur Energieerzeugung genutzt werden sollen, benötigen üblicherweise eine Erhöhung der Energiedichte. Dazu ist es erforderlich, unbrennbare Inertgase aus dem Gasgemisch zu entfernen.

Tiefkalt verflüssigtes Methan (LNG) zum Beispiel besitzt ein wachsendes Marktpotential, da es die maximale Energiedichte dieses Energieträgers darstellt und einen kostengünstigen Transport ermöglicht. LNG findet Anwendung als Energieträger zum Betreiben von Blockheizkraftwerken oder als Kfz-Treibstoff. Es kann sowohl aus Erdgas als auch aus biologisch-mechanischer Verwertung von nachwachsenden Rohstoffen und Abfällen gewonnen werden und stellt dadurch einen Energieträger dar, der unabhängig von fossilen Ressourcen hergestellt werden kann.

Beispiele hierfür sind Biogas oder Klärgas, welche neben Methan eine hohe Kohlendioxidkonzentration enthalten. Aus diesem Grund wird besonderes Augenmerk auf die Abtrennung des Kohlendioxids von methanhaltigen Gemischen gelegt.

Bekannte Verfahren zur Abtrennung des Kohlendioxids aus Gemischen beziehen sich auf die Adsorption des Kohlendioxids an ein spezielles Adsorbens, die Gaswäsche mit Wasser oder Chemikalien, die Permeation durch Spezialmembranen bzw. Molsiebe oder die TieftemperaturDestillation mit Verflüssigung des Kohlendioxids. Diese Verfahren und Vorrichtungen zur Erzeugung von Trockeneis nutzen allesamt die Abkühlung eines Gases bei der adiabatischen Expansion (Joule-Thomson-Effekt) von gasförmigem oder flüssigem Kohlendioxid, das unter einem Druck von ca. 55 bar steht und auf ca. 4,8 bar oder Atmosphärendruck entspannt wird. Die Entspannung des Gases erfolgt meist in einer Düse mit besonderer Geometrie, um einen Teil des desublimierten Kohlendioxids in Form von Schnee zu erhalten. Je nach Aufgabenstellung wird der erzeugte Kohlendioxidschnee durch verschiebbare Kolben zu Trockeneistabletten oder -scheiben gepresst und zur Weiterverwendung ausgeworfen. Den bisherigen Verfahren ist gemein, dass der optimierte Gasreinigungsprozess bei erhöhtem Druck stattfindet, wobei die Kompression des Gases den größten Anteil der benötigen Leistung verursacht. Ferner werden durch die Prozessführung bei großem Überdruck aufwendigere Armaturen und Leitungen erforderlich, wodurch höhere Kosten für Investitionen und Energie zustande kommen.

Ein weiteres Verfahren zur Herstellung von Trockeneis mit bestimmten Eigenschaften ist in der Auslegeschrift DE 1 181 186 beschrieben. Hierin wird die gasförmige Kohlensäure mit einem Gehalt von nicht kondensierbaren Gasen von weniger als 25 Vol.-% in einem kontinuierlichen Strom über die Kühlfläche geführt. Die Temperatur der Kühlfläche ist höchstens 25 °C niedriger als die dem Partialdruck der Kohlensäure entsprechende Sublimationstemperatur, damit das Trockeneis mit den gewünschten Eigenschaften hergestellt werden kann.

Außerdem findet Trockeneis in industriellen und Lebensmittel erzeugenden Branchen zur Kühlung Verwendung und wird als rückstandfreies Strahlmittel zur Entfernung von Beschichtungen eingesetzt.

Die Aufgabe der vorliegenden Erfindung besteht in der Abtrennung von Methan aus methanhaltigen Gemischen bei geringerem Energieaufwand.

Diese Aufgabe wird durch die vorliegende Erfindung gelöst, indem das abgetrennte, gasförmige Methan aus dem Auslass einer Abtrennkammer zusammen mit dem verdampften Methan aus den Wärmetauschern, welche sich in der Abtrennkammer befinden, zumindest teilweise in eine Verflüssigungseinheit geführt und dort verflüssigt wird. Das somit verflüssigte Methan wird in den Wärmetauschern der Abtrennkammer als Kühlmittel benutzt. Optional kann das verflüssigte Methan in einen Tank geleitet und dort gelagert werden. Weiter optional kann das gasförmige, gereinigte Methan in ein Gasnetz eingespeist werden. Außerdem kann aus methanhaltigen Gemischen, welche eine hohe Konzentration von Kohlendioxid enthalten, Trockeneis gewonnen werden und als Kühlmittel eines Wärmetauschers einer Vorbehandlungseinrichtung eingesetzt werden.

Ein Vorteil der vorliegenden Erfindung liegt darin, dass ein Großteil der zur Gasaufbereitung eingesetzten Energie bei der Gasverflüssigung und/oder der Gasreinigung eingespart werden kann.

Gemäß dieser Erfindung werden die folgenden Begriffe definiert:

Ein methanhaltiges Gemisch ist eine Gasmischung, welche Methan und unbrennbare Inertgase enthält. Beispiele von methanhaltigen Gemischen sind Biogas, Klärgas, Faulgas und Grubengas.

Desublimation ist der Phasenübergang, welcher von dem gasförmigen Zustand direkt zu dem festen Zustand einer Substanz führt.

Ausfrieren einer Komponente eines Gasgemisches umfasst gemäß dieser Erfindung alle Phasenübergänge, welche zu einem festen Zustand der Komponente führen, sowie den Phasenübergang von gasförmig zu flüssig, also Kondensation, Gefrieren und Desublimation.

Eine erfindungsgemäße Vorrichtung zur Abtrennung von Methan aus methanhaltigen Gemischen, deren Komponenten eine höhere Siede-, Schmelz- oder Sublimationstemperatur als das flüssige Methan aufweisen, weist mindestens einen Wärmetauscher mit einem Kühlmitteleinlass und einem Kühlmittelauslass, eine Abtrennkammer, welche den mindestens einen Wärmetauscher umschließt und einen Auslass für das abgetrennte, gasförmige Methan aufweist, eine Verflüssigungseinheit, eine Leitung für das verflüssigte Methan und eine Leitung für das gasförmige Methan auf. Erfindungsgemäß verbindet die Leitung für das verflüssigte Methan die Verflüssigungseinheit mit dem mindestens einen Wärmetauscher und die Leitung für das gasförmige Methan die Verflüssigungseinheit sowohl mit dem mindestens einen Wärmetauscher als auch mit dem Auslass für das abgetrennte, gasförmige Methan der Abtrennkammer.

Eine erfindungsgemäße Vorrichtung kann mehr als nur einen Wärmetauscher aufweisen. Dann werden die Auslässe für das gasförmige Methan der mehreren Wärmetauscher zusammengefasst und mit der Verflüssigungseinheit verbunden.

Wenn das methanhaltige Gemisch Kohlendioxid in hoher Konzentration enthält, kann dieses auf der Oberfläche jedes Wärmetauschers durch das Ausfrieren gewonnen werden. Aufgrund des hohen Ausdehnungskoeffizienten von Trockeneis bilden sich bei dessen Abkühlung Risse in der auf der Oberfläche der Wärmetauscher ausgefrorenen Schicht. Diese Risse erleichtern das Ablösen des Trockeneises von den Wärmetauschern. Somit kann neben der Reinigung und/oder Abtrennung des Methans auch Trockeneis erzeugt werden. Außerdem kann das Trockeneis vorteilhaft als Kühlmittel in einem Wärmetauscher, welcher zu einer Vorbehandlungseinrichtung gehört, weiter verwendet werden.

In einer bevorzugten Ausführungsform der Vorrichtung ist jeder der Wärmetauscher als Plattenwärmetauscher ausgeführt. Vorteilhaft ist dabei, wenn die Platten jedes Wärmetauschers eine sehr glatte Oberfläche aufweisen, um das Ablösen der ausgefrorenen Komponenten des methanhaltigen Gemisches von den Wärmetauschern zu erleichtern.

In einer weiteren bevorzugten Ausführungsform der Vorrichtung sind die Wärmetauscheroberflächen mit einer Beschichtung versehen, an der das Trockeneis nur in geringem Maße anhaftet.

Besonders vorteilhaft ist es, wenn die Wände der Wärmetauschers aus Edelstahlblech (insbesondere aus hochlegiertem, kaltzähem Edelstahl) sind und sich konvex nach außen wölben, sobald sich im Inneren ein Druck aufbaut.

In einer weiteren bevorzugten Ausführungsform der Vorrichtung weist mindestens einer der Wärmetauscher ein Heizelement auf. Ein Heizen des Wärmetauschers erleichtert das Ablösen der am Wärmetauscher ausgefrorenen Komponenten des methanhaltigen Gemisches, zum einen durch die Sublimation der untersten Lage der ausgefrorenen Schicht, zum anderen dadurch, dass der Wärmetauscher mittels Ventilen in der Leitung für das flüssige Methan als auch in der Leitung für das gasförmige Methan von dem übrigen Leitungssystem getrennt werden kann und das im Wärmetauscher befindliche Methan durch die Erwärmung expandiert und somit den Druck im Wärmetauscher erhöht, was zu einer Wölbung der Oberfläche des Wärmetauschers nach außen führt.

In einer weiteren bevorzugten Ausführungsform weist die Vorrichtung ein Austragssystem auf, in dem die abgelösten ausgefrorenen Komponenten des methanhaltigen Gemisches aufgefangen und gesammelt werden. Bevorzugterweise weist das Austragssystem einen Auffangtrog, einen Kolben, welcher mittels eines Hydrauliksystems betätigt wird, einen Schieber und eine Austragsleitung auf. Von besonderem Vorteil ist, dass die erfindungsgemäße Vorrichtung über die Austragsleitung des Austragssystems an einer Vorbehandlungseinrichtung mittels eines Wärmetauschers zur Rückgewinnung der Kälte von den ausgefrorenen Komponenten des methanhaltigen Gemisches angeschlossen ist.

In einer bevorzugten Ausführungsform besteht der Wärmetauscher zur Rückgewinnung der Kälte aus den ausgefrorenen Komponenten des methanhaltigen Gemisches aus einem Behälter, aus einer Leitung für den Rücklauf des Kühlmittels, aus einem Rost und Gittern, welche eine Schüttung der ausgefrorenen Komponenten des methanhaltigen Gemisches halten und welche die Befüllung von Kühlmittel in die Zwischenräume der Schüttung erlaubt, aus einer Leitung für den Vorlauf des kalten Kühlmittels, aus einer Leitung für den Vorlauf der bei der Abkühlung des Kühlmittels sublimierten ausgefrorenen Komponenten des methanhaltigen Gemisches und aus einer Zuführleitung für die ausgefrorenen Komponenten des methanhaltigen Gemisches, welche von der Austragsleitung abzweigt. Besonders vorteilhaft ist die Leitung für den Rücklauf des Kühlmittels an dem unteren Teil des Behälters angeschlossen, während die Leitung für den Vorlauf der sublimierten ausgefrorenen Komponenten des methanhaltigen Gemisches und des Kühlmittels an dem oberen Teil des Behälter angeschlossen sind. Vorzugsweise wird als Kühlmittel ein Alkohol benutzt, besonders bevorzugt ist Ethanol. Auch der Einsatz von Methanol, Propanol oder Butanol als Kühlmittel ist möglich.

In einer bevorzugten Ausführungsform weist die Vorbehandlungseinrichtung eine Leitung auf, welche das methanhaltige Gemisch durch zwei Wärmetauscher zu der Abtrennkammer führt. Die zwei Wärmetauscher befinden sich in einem gemeinsamen Kühlkreislauf mit dem oben beschriebenen Wärmetauscher zur Rückgewinnung des Kälte aus den ausgefrorenen Komponenten des methanhaltigen Gemisches. In dem Kühlkreislauf sind zwei Ventile vorgesehen, die entgegengesetzt geschaltet werden können, so dass das Kühlmittel nur einen der beiden Wärmetauscher durchströmt.

Vorteilhafterweise ist die Leitung, durch die das methanhaltige Gemisch durch zwei Wärmetauscher zu der Abtrennkammer geführt wird, eine Leitungsschlaufe mit zwei möglichen Gaswegen, welche in unterschiedlichen Strömungsrichtungen betrieben werden können. Die Leitungsschlaufe hat zwei Einlässe für das ungekühlte methanhaltige Gemisch und einen Auslass für das vorgekühlte methanhaltige Gemisch. Die Vorbehandlungseinrichtung weist zwei Ventile auf, von denen jeweils eines an der Zuleitung kurz vor den Einlässen der Leitungsschlaufe angeordnet ist und die entgegengesetzt geschaltet werden können, so dass das ungekühlte methanhaltige Gemisch nur über einen Einlass in die Leitungsschlaufe geleitet wird.

Weiterhin weist die Leitungsschlaufe der Vorbehandlungseinrichtung zwei Ventile auf, wobei das erste Ventil zwischen dem ersten Einlass für das ungekühlte methanhaltige Gemisch und dem Auslass für das vorgekühlte methanhaltige Gemisch angeordnet ist und das zweite Ventil zwischen dem zweiten Einlass für das ungekühlte methanhaltige Gemisch und dem Auslass für das vorgekühlte methanhaltige Gemisch angeordnet ist. Auch diese beiden Ventile können entgegengesetzt geschaltet werden, wodurch die Strömungsrichtung durch die Leitungsschlaufe umgekehrt werden kann.

In einer weiteren bevorzugten Ausführungsform weist die erfindungsgemäße Vorrichtung einen Tank zum Sammeln des flüssigen Methans auf, wobei der Tank mit der Leitung für das verflüssigte Methan, die die Verflüssigungseinheit mit dem Kühlmitteleinlass der Wärmetauscher in der Abtrennkammer verbindet, verbindbar ist. Bevorzugterweise ist der Tank mit dieser Leitung mittels eines Drei-Wege-Reglers verbindbar. In dem Tank kann das flüssige Methan gesammelt werden und für weitere Verwendungen bereitgestellt werden.

In einer weiteren bevorzugten Ausführungsform weist die erfindungsgemäße Vorrichtung einen Anschluss an ein Gasnetz, beispielsweise ein öffentliches Gasnetz, auf. Bevorzugt ist dieser Anschluss mit der Leitung für das gasförmige Methan zwischen der Verflüssigungseinheit und der Abtrennkammer verbunden oder in dieser angeordnet. Dieser Anschluss an ein Gasnetz erlaubt es, abgetrenntes und damit gereinigtes gasförmiges Methan in das Gasnetz einzuspeisen. Bevorzugt ist dieser Anschluss als Drei-Wege-Regler ausgeführt, der in der Leitung für das gasförmige Methan angeordnet ist, wobei die Abtrennkammer in einer Stellung des Drei-Wege-Reglers mit der Verflüssigungseinheit verbunden ist und in einer anderen Stellung des Drei-Wege-Reglers mit dem Gasnetz verbunden ist. Zwischen Drei-Wege-Regler und Gasnetz kann noch ein Kompressor angeordnet sein, um das gasförmige Methan auf einen im Gasnetz vorhandenen Druck (üblicherweise zwischen 6 bar und 90 bar) zu komprimieren.

Bevorzugte Konzentrationen von Restsubstanzen in dem gasförmigen Methan, welches aus dem Auslass der Abtrennkammer ausströmt, sind niedriger als 5 %, besonders bevorzugt niedriger als 3 %.

Methanhaltige Gemische, für die die Vorrichtung gemäß der vorliegenden Erfindung besonders geeignet ist, enthalten als zweiten Hauptbestandteil Kohlendioxid, wobei die Konzentration des Kohlendioxids nicht höher ist als 50 Vol.-%, besonders bevorzugt ist eine Konzentration des Kohlendioxids von 40 Vol.-%. Üblicherweise haben Biogase eine Zusammensetzung zwischen [65 % Methan + 30 % CO₂] und [50 % Methan + 45 % CO₂].

Diese Erfindung betrifft des Weiteren ein Verfahren zur Abtrennung von Methan aus methanhaltigen Gemischen, deren Komponenten eine höhere Siede-, Schmelz- oder Sublimationstemperatur als das flüssige Methan aufweisen, wobei das methanhaltige Gemisch über einen Einlass in eine Abtrennkammer mit mindestens einem Wärmetauscher geleitet wird und das abgetrennte Methan über einen Auslass aus der Abtrennkammer geleitet wird. Erfindungsgemäß wird das abgetrennte Methan aus dem Auslass der Abtrennkammer zusammen mit verdampften Methan aus dem mindestens einem Wärmetauscher zumindest teilweise in eine Verflüssigungseinheit geleitet und in der Verflüssigungseinheit verflüssigt und das verflüssigte Methan wird aus der Verflüssigungseinheit in den mindestens einen Wärmetauscher geleitet.

In einer bevorzugten Ausführungsform des Verfahrens überströmt das methanhaltige Gemisch einen oder mehrere Wärmetauscher, bevorzugt Plattenwärmetauscher. Vorteilhaft werden die Plattenwärmetauscher kontinuierlich mit verflüssigtem Methan als Kühlmittel mit einer Temperatur von etwa-162 °C befüllt.

In einer weiteren bevorzugten Ausführungsform des Verfahrens gemäß der Erfindung wird zumindest ein Teil des verflüssigten Methans in einen Tank geleitet, der mit der Leitung für das verflüssigte Methan, die die Verflüssigungseinheit mit dem Kühlmitteleinlass der Wärmetauscher in der Abtrennkammer verbindet, verbunden ist. Bevorzugterweise erfolgt die Verbindung des Tanks mit dieser Leitung mittels eines Drei-Wege-Reglers. In dieser Ausführungsform kann mit dem erfindungsgemäßen Verfahren flüssiges Methan hergestellt werden.

In einer weiteren bevorzugten Ausführungsform des Verfahrens wird nur der Anteil des abgetrennten Methans in die Verflüssigungseinheit geleitet, der benötigt wird, um durch die Gastrennung verdampftes Methan aus dem mindestens einem Wärmetauscher durch flüssiges Methan als Kühlmittel zu ersetzen. Der Anteil des abgetrennten, gasförmigen Methans, der nicht als Ersatz für das Kühlmittel benötigt wird, kann einer anderweitigen Verwendung zugeführt werden. In dieser Ausführungsform kann mit dem erfindungsgemäßen Verfahren gasförmiges Methan erzeugt werden.

Es ist mit dem erfindungsgemäßen Verfahren ebenfalls möglich, sowohl flüssiges als auch gasförmiges Methan zu erzeugen, indem ein erster Anteil des abgetrennten Methans noch gasförmig einer anderweitigen Verwendung zugeführt wird und ein zweiter Anteil des abgetrennten Methans zusammen mit dem verdampften Methan aus dem mindestens einen Wärmetauscher in der Verflüssigungseinheit verflüssigt wird, wobei ein erster Anteil des verflüssigten Methans als Ersatz für verdampftes Kühlmittel verwendet wird und der verbleibende zweite Anteil des verflüssigten Methans in einen Tank geleitet wird.

In einer weiteren bevorzugten Ausführungsform des Verfahrens wird zumindest ein Teil des abgetrennten Methans aus dem Auslass der Abtrennkammer zusammen mit verdampften Methan aus dem mindestens einem Wärmetauscher in ein Gasnetz (beispielsweise ein öffentliches Gasnetz) eingespeist, das mit der Leitung für das gasförmige Methan beispielsweise über einen Drei-Wege-Regler verbunden ist. Hierzu kann das gasförmige Methan, falls erforderlich, mit einem Kompressor auf einen im Gasnetz vorhandenen Druck (üblicherweise zwischen 6 bar und 90 bar) komprimiert werden.

Durch das Überströmen der Wärmetauscher wird das methanhaltige Gemisch bis zur Kondensations-, Sublimations- oder Schmelztemperatur der weiteren Komponenten des methanhaltigen Gemisches abgekühlt. Dadurch frieren diese Komponenten an den Oberflächen der Wärmetauscher aus und werden danach von der Oberfläche des Wärmetauschers abgelöst.

In einer bevorzugten Ausführungsform des Verfahrens erfolgt das Ablösen der an den Oberflächen der Wärmetauscher ausgefrorenen Komponenten des methanhaltigen Gemisches von der Oberfläche der Wärmetauscher dadurch, dass die aus diesen Komponenten bestehende Schicht aufgrund ihres hohen Volumenaus dehnungs koeffizienten beim Abkühlen ihr Volumen verringert und dadurch Risse bekommt und/oder abplatzt.

In einer weiteren bevorzugten Ausführungsform des Verfahrens erfolgt das Ablösen der an den Oberflächen der Wärmetauscher ausgefrorenen Komponenten des methanhaltigen Gemisches von der Oberfläche der Wärmetauscher durch Erwärmen des Wärmetauschers, beispielsweise mittels eines in den Wärmetauscher integrierten Heizelements. Dadurch sublimiert oder schmilzt die unterste Schicht der ausgefrorenen Komponenten und die oberen Schichten lösen sich von der Oberfläche der Wärmetauscher.

In einer weiteren bevorzugten Ausführungsform des Verfahrens erfolgt das Ablösen der an den Oberflächen der Wärmetauscher ausgefrorenen Komponenten des methanhaltigen Gemisches von der Oberfläche der Wärmetauscher dadurch, dass die Oberfläche der Wärmetauscher durch Erhöhen des Drucks im Inneren des Wärmetauschers nach außen gedrückt wird. In einer besonders bevorzugten Ausführungsform erfolgt das Erhöhen des Drucks im Inneren der Wärmetauscher durch druckdichtes Abschließen der Wärmetauscher gegenüber dem mit den Wärmetauschern verbundenen Leitungssystem und durch Erwärmen des Methans im Inneren der Wärmetauscher.

Bevorzugte Konzentrationen von Restsubstanzen in dem gasförmigen Methan, welches aus dem Auslass der Abtrennkammer ausströmt, sind niedriger als 5 %, besonders bevorzugt niedriger als 3 %.

In einer weiteren bevorzugten Ausführungsform des Verfahrens werden die ausgefrorenen Komponenten in einem Auffangtrog aufgefangen. In einer besonders bevorzugten Ausführungsform werden die in dem Auffangtrog aufgefangenen ausgefrorenen Komponenten von einem Kolben, der bevorzugterweise hydraulisch, pneumatisch oder elektrisch angetrieben wird, aus dem Auffangtrog in eine Austragsleitung geschoben. Die Austragsleitung kann einen Schieber aufweisen, gegen den die ausgefrorenen Komponenten gepresst werden, um sie zu verdichten. Der Schieber kann periodisch oder in unregelmäßigen Zeitabständen geöffnet werden, damit die ausgefrorenen Komponenten weiter in die Austragsleitung geschoben werden können.

Bevorzugterweise weist das verwendete methanhaltige Gemisch eine hohe Konzentration von Kohlendioxid auf, welches aus dem methanhaltigen Gemisch zu der Desublimation von Kohlendioxid führt und somit zur Herstellung von Trockeneis geeignet ist.

Methanhaltige Gemische, für die das Verfahren gemäß der vorliegenden Erfindung besonders geeignet ist, enthalten eine Konzentration des Kohlendioxids von nicht höher als 50 Vol-%, besonders bevorzugt ist eine Konzentration des Kohlendioxids von 40 Vol.-%.

In einer weiteren bevorzugten Ausführungsform des erfindungsgemäßen Verfahrens wird das methanhaltige Gemisch durch die Kälteleistung des während des Verfahrens erzeugten Trockeneises vorgekühlt und entfeuchtet. Dies ist besonders vorteilhaft, da viele methanhaltige Gasgemische, insbesondere Biogase einen hohen Anteil an Wasser aufweisen. Wasserdampf, der in die Abtrennungskammer gelangt wirkt sich störend auf den Prozess aus, weil der Wasserdampf durch seinen hohen Gefrierpunkt beim Anströmen der Plattenwärmetauscher sofort gefriert und dort zu einer starken Vereisung führt, was die gleichmäßige Durchströmung der Abtrennungskammer behindert. Außerdem lässt sich Wassereis - wegen der hohen Schmelztemperatur und dem geringen Wärmeausdehnung (Volumenausdehnungskoeffizient α = 1,506 · 10⁻⁶ K⁻¹) - nicht durch die für das Trockeneis vorgesehenen Abreinigungsmechanismen ablösen.

In einer weiteren bevorzugten Ausführungsform des erfindungsgemäßen Verfahrens wird das methanhaltige Gemisch dadurch vorgekühlt und entfeuchtet, dass es über mindestens einen Wärmetauscher geleitet wird, dessen Kühlflüssigkeit durch die Kälteleistung des erzeugten Trockeneises gekühlt wird.

In einer weiteren bevorzugten Ausführungsform des erfindungsgemäßen Verfahrens wird das methanhaltige Gemisch dadurch vorgekühlt und entfeuchtet, dass es durch eine Leitungsschlaufe geleitet wird, die mit zwei Wärmetauschern in thermischem Kontakt steht, wobei die Kühlflüssigkeit des einen Wärmetauschers durch die Kälteleistung des erzeugten Trockeneises gekühlt wird und der andere Wärmetauscher durch das methanhaltige Gemisch erwärmt wird. Besonders bevorzugt wird, dass die Strömungsrichtung des methanhaltigen Gemisches durch die Leitungsschlaufe umgekehrt werden kann. Die Umkehrung der Strömungsrichtung erfolgt vorzugsweise in Verbindung mit dem Umschalten des Kühlkreislaufs von einem Wärmetauscher auf den anderen, um den zunächst zum Kühlen verwendeten Wärmetauscher, an dem das Wasser aus dem methanhaltigen Gemisch ausgefroren ist, mit dem warmen methanhaltigen Gemisch abzutauen. Das hierbei entstehende Wasser verlässt die Vorrichtung über einen Austragssiphon.

Gemäß der Erfindung wird vorzugsweise das methanhaltige Rohgemisch, d.h. das noch unbehandelte methanhaltige Gemisch, in die Vorbehandlungseinrichtung und durch einen ersten Wärmetauscher geleitet. Dieser erste Wärmetauscher kann von dem Vorzyklus an seinen Außenwände vereist sein und wird nicht vom durch das Trockeneis gekühlten Kühlmittel durchströmt. Durch das Umströmen des warmen methanhaltigen Rohgemisches taut das Wasser auf und wird über einen Austragssiphon entfernt, während das methanhaltigen Rohgemisch abgekühlt wird. Das methanhaltige Rohgemisch strömt weiter über den zweiten Wärmetauscher, welcher vom durch das Trockeneis gekühlten Kühlmittel durchströmt wird. Dabei friert das Wasser, das in dem methanhaltigen Rohgemisch enthalten ist, auf den Außenwänden des zweiten Wärmetauschers der Vorbehandlungseinrichtung aus. Das abgekühlte methanhaltige Gemisch verlässt die Vorbehandlungseinrichtung und wird vorzugsweise in die Abtrennkammer geleitet.

Die Kühlung der Kühlflüssigkeit durch die Kälteleistung des erzeugten Trockeneises erfolgt in einer weiteren bevorzugten Ausführungsform des erfindungsgemäßen Verfahrens dadurch, dass das Kühlmittel durch einen Behälter geleitet wird, welcher mit einer Schüttung aus Trockeneis beschickt ist. Das Kühlmittel kommt dabei mit dem Trockeneis optimal in Kontakt. Das bei diesem Vorgang sublimierte Kohlendioxid wird aus dem Behälter geleitet und kann weiterhin für Kühlzwecke - auch im Rahmen des erfindungsgemäßen Verfahrens zur weiteren Vorkühlung des methanhaltigen Gemisches - eingesetzt werden.

Vorzugsweise strömt das Kühlmittel durch einen Rücklauf in einen Behälter, welcher mit einer Schüttung aus Trockeneis beschickt ist, wobei die Schüttung auf einem Rost liegt und von Gittern festgehalten ist. Das Kühlmittel fließt durch den Zwischenraum in der Trockeneisschüttung und gibt dadurch Wärme an das Trockeneis ab und kühlt somit ab. Das kalte Kühlmittel fließt über einen Vorlauf wieder in den Kreislauf der Vorbehandlungseinrichtung.

Bevorzugterweise wird ein Alkohol als Kühlmittel benutzt, um die Wärmetauscher zum Vorkühlen und Entfeuchten des methanhaltigen Gemisches zu kühlen. Besonders bevorzugt ist hierbei der Einsatz von Ethanol. Auch der Einsatz von Methanol, Propanol oder Butanol als Kühlmittel ist möglich.

In einer weiteren bevorzugten Ausführungsform des erfindungsgemäßen Verfahrens wird als Stoffstrompumpe die Partialdruckdifferenz des methanhaltigen Rohgemisches am Einlass der Abtrennkammer zu dem abgetrennten Methan am Auslass der Abtrennkammer verwendet. Dadurch ist es nicht erforderlich, zusätzliche Pumpen einzusetzen, die die Vorrichtung wartungsanfälliger und teurer machen und zusätzliche Energie verbrauchen. Zum Ausgleich von Rohrleitungsverlusten kann allerdings auch eine Pumpe oder ein Gebläse zum Einsatz kommen, die/das den Transport des methanhaltigen Gemisches durch die Vorrichtung unterstützt.

Idealerweise wird das erfindungsgemäße Verfahren unter Atmosphärendruck durchgeführt. Es kann aber auch unter Überdruck durchgeführt werden, wobei der Druck in der Abtrennkammer der Vorrichtung dabei nicht über dem Tripelpunkt der auszufrierenden Komponente liegen sollte, um die Desublimation dieser Komponente sicherzustellen. Insbesondere wenn die auszufrierende Komponente Kohlendioxid ist, sollte der Druck in der Abtrennkammer daher nicht über 5 bar liegen.

Anhand der Zeichnungen wird die Erfindung nachstehend eingehend erläutert. Es zeigt:
Fig. 1 eine erfindungsgemäße Vorrichtung zur Abtrennung von Methan aus methanhaltigen Gasgemischen einschließlich einer geschnittenen Ansicht der Abtrennkammer mit der um 90° gedrehten Wärmetauscherpackung;
Fig. 2 eine Vorbehandlungsanlage zur erfindungsgemäßen Vorbehandlung des methanhaltigen Gasgemisches; und
Fig. 3 einen Wärmetauscher zur Rückgewinnung der Kälte aus dem Trockeneis.

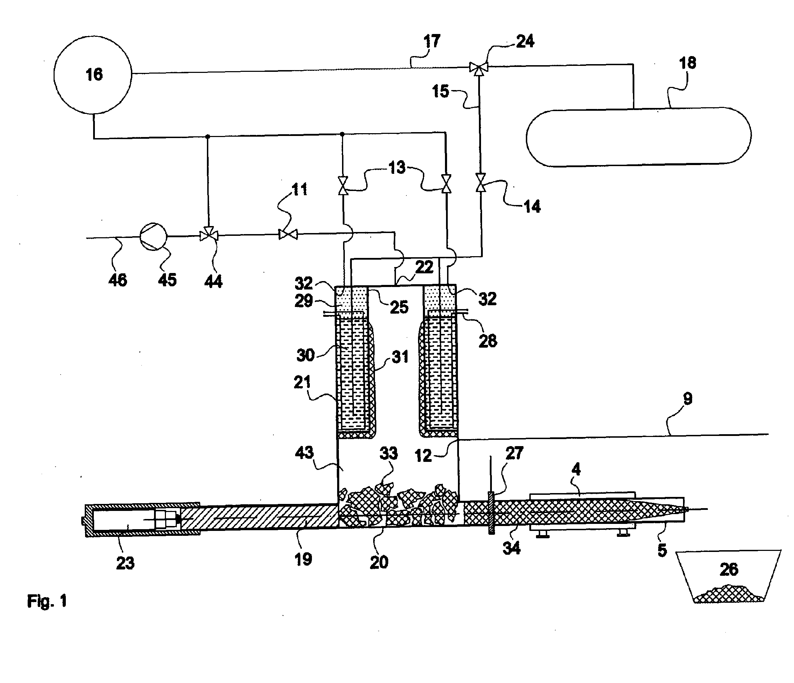

In der Darstellung gemäß Fig. 1 ist eine erfindungsgemäße Vorrichtung zur Durchführung des erfindungsgemäßen Verfahrens gezeigt. Die gezeigte Vorrichtung ist geeignet für die Abtrennung von Methan aus einem methanhaltigem Gasgemisch welches als weitere Komponente Kohlendioxid enthält. Somit kann die dargestellte Vorrichtung auch zur Erzeugung von Trockeneis genutzt werden.

Durch die Leitung (9) und durch mehrere Bohrungen (12) durchströmt das methanhaltige Gemisch die Abtrennkammer (43), in welcher zwei Wärmetauscher (21) angeordnet sind, bevor es durch Auslassöffnungen (22), welche sich zwischen den Wärmetauschern (21) befinden, die Abtrennkammer (43) verlässt. Durch das Überströmen der Wärmetauscher, die mit flüssigem Methan gekühlt werden, kühlt das methanhaltige Gemisch bis unter der Sublimationstemperatur des Kohlendioxids ab, welches somit an der Oberfläche der Wärmetauscher (21) desublimiert. Die Volumenverringerung durch die Abkühlung des methanhaltige Gemisches und den Phasenübergang des Kohlendioxids vom Gas zum Festkörper erzeugt einen Unterdruck, der genau die Menge an methanhaltigen Gemisch ansaugt, die an den Wärmetauscheroberflächen gereinigt werden kann. Die Wärmetauscher (21) werden kontinuierlich mit verflüssigtem Methan (30) befüllt, das als Kühlmittel für die Wärmetauscher bei einer Temperatur von etwa -162 °C verwendet wird.

Die in diesem Prozessschritt übertragene Wärme führt zur Verdampfung des verflüssigten Methans im Innenraum der Wärmetauscher, wobei das verflüssigte Methan als verdampftes Boil-off-Gas durch die Auslassöffnungen (32) abströmt. Durch eine Regelung des Drei-Wege-Reglers (24) strömt stets so viel verflüssigtes Methan in den Innenraum der Wärmetauscher, wie zur Gastrennung, d.h. zum Ersetzen des Boil-off-Gas benötigt wird.

Das gasförmige Frisch-Methangas, d.h. das in der Abtrennkammer gereinigte Methan, strömt durch die am oberen Ende der Abtrennkammer angebrachten Auslassöffnungen (22) aus der Abtrennkammer und wird von der Verflüssigungseinheit (16) angesaugt und durch Abkühlen verflüssigt. Außerdem saugt die Verflüssigungseinheit das verdampfte Methangas (29) aus den Wärmetauschern (21) an und verflüssigt es. Das erzeugte verflüssigte Methan strömt über die Leitung (15) in die Wärmetauscher (21), wobei der verflüssigte Methanrücklauf über einen Drei-Wege-Regler (24) bedarfsabhängig geregelt wird und das nicht im Prozess benötigte verflüssigte Methan durch die Leitung (17) in den Tank (18) geleitet wird.

In der Leitung für das gasförmige Methan von der Abtrennkammer (43) zur Verflüssigungseinheit (16) befindet sich ein Drei-Wege-Regler (44). Dieser verbindet in einer ersten Stellung die Abtrennkammer (43) mit der Verflüssigungseinheit (16), sodass das abgetrennte, gereinigte Methan wie oben beschrieben von der Verflüssigungseinheit (16) angesaugt wird. In einer zweiten Stellung des Drei-Wege-Reglers (44) ist die Verbindung zwischen Abtrennkammer (43) und Verflüssigungseinheit (16) gesperrt und das gasförmige Methan aus der Abtrennkammer wird statt dessen von einem Kompressor (45) angesaugt und komprimiert. Das komprimierte gasförmige Methan wird dann über die Leitung (46) in das öffentliche Gasnetz eingespeist.

Bei der Abtrennung des Kohlendioxids vom methanhaltigen Gemisch baut sich an der Oberfläche der Wärmetauscher (21) eine Trockeneisschicht (31) auf, die periodisch abgereinigt werden muss. Bei der Abkühlung des Trockeneis um ca. 30 °C kommt es durch das Schrumpfen des Trockeneis (Volumenausdehnungskoeffizient α = 76·10⁻⁵ K⁻¹, bei T = 173 K) zu Dehnungsspannungen der Trockeneissicht (31), die zum Zerbrechen derselben führen. Das Ablösen des Trockeneises wird durch die besonders niedrige Rauheit der Wärmetauscheroberfläche (25) gefördert.

Eine elektrische Heizung (28), die im Innenraum des Wärmetauschers angeordnet ist, unterstützt das Ablösen des Trockeneises durch zwei Mechanismen. Das Aktivieren der Heizung führt zum Verdampfen des verflüssigten Methans im Inneren des Wärmetauschers zu Methangas, das bei geschlossenem Ventil (13) das verflüssigte Methan in die Zuführleitung (15) zurückdrückt, bis ausschließlich gasförmiges Methan in dem abzutauenden Wärmetauscher befindet. Nach dem Absperren des Ventils (14) der verflüssigten Methanzuleitung führt die Wärmeausdehnung des gasförmigen Methans zum Ausbeulen der Wärmetauscheroberfläche (25) und somit zum Ablösen der Trockeneisschichten (31). Außerdem wird anhaftendes Trockeneis im unteren Teil der Schicht durch die Heizwärme verdampft, wodurch das Ablösen weiter unterstützt wird. Während des Abtauvorgangs wird das Ventil (11) abgesperrt, da das Gas nicht ausreichend getrennt wird.

Die abgelösten Trockeneisbrocken (33) fallen in den Auffangtrog (20) des Austragssystems, das sich mindestens so weit unterhalb der Unterkante der Wärmetauscher befindet, um die vollständige Entfernung der Trockneiseisschichten aus den Zwischenräumen der Wärmetauscher zu gewährleisten.

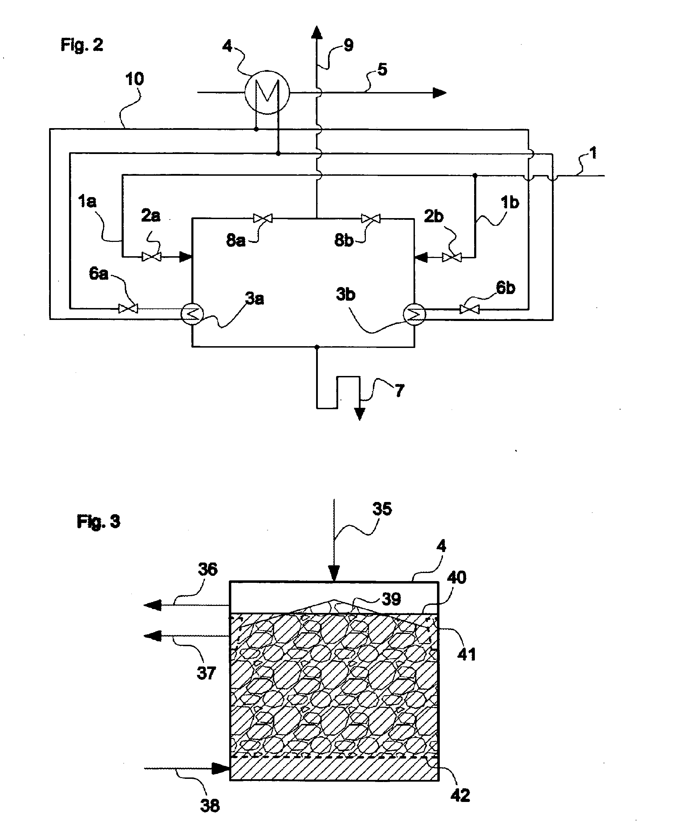

Die Trockeneisbrocken werden von einem Kolben (19), der von einem Hydrauliksystem (23) angetrieben wird, periodisch aus dem Auffangtrog (20) entfernt, wobei das Trockeneis zuerst gegen einen geschlossenen Schieber (27) gepresst wird. Sobald sich ein kompakter Trockeneispfropf (34) gebildet hat, der die Austragsleitung abdichtet, wird der Schieber (27) dauerhaft geöffnet und der Trockeneispfropf wird in die Austragsleitung (5) geschoben, welche die Vorbehandlungseinrichtung (in Fig. 2 dargestellt) mit Trockeneis versorgt. Das Ausströmen des zu trennenden methanhaltigen Gasgemisches wird durch den kompakten Trockeneispfropf verhindert, der die Austragleitung (5) abdichtet.

Eine bevorzugte Vorrichtung des Wärmetauschers (4) zur Rückgewinnung der Kälte aus dem Trockeneis besteht aus einem Behälter, welcher mit einer Schüttung (39) aus Trockeneis beschickt wird, wobei das Trockeneis von der Austragsleitung (5) abgezweigt und über die Zufuhrleitung (35) in den Behälter eingebracht wird. Der Wärmetauscher (4) wird über einen Rücklauf (38) mit einem Kühlmittel befüllt, wobei das Kühlmittel durch das Fließen in den Zwischenraum der Trockeneisschüttung (39) die Kälte aufnimmt und dadurch abgekühlt wird. Das kalte Kühlmittel fließt über einen Vorlauf (37) wieder in den Kühlkreislauf (10). Das bei der Abkühlung des Kühlmittels sublimierte Kohlendioxid wird über einen Vorlauf (36) abgeführt und ebenfalls zur Vorbehandlung des Gasgemisches verwendet. Die Schüttung (39) liegt auf einem Rost (42) und wird von Gittern (41), die sich über dem Vorlauf (37) befinden, im Wärmetauscher (4) festgehalten.

Durch die Vorbehandlung des methanhaltigen Gasgemisches sublimiert ein Teil des Trockeneises und verlässt das System gasförmig, während das restliche Trockeneis im festen Zustand aus dem Prozess ausgetragen wird. Dieses Kohlendioxid wird in einen Sammelbehälter (26) abgeworfen und steht einer Weiterverwendung zu Verfügung.

Die Vorbehandlungseinrichtung aus Fig. 2 ist eine Schlaufe mit zwei möglichen Gaswegen, die in unterschiedlichen Strömrichtungen betrieben werden kann und durch Umschalten des Gasweges ein periodisches Abtauen ermöglicht.

Die beiden Betriebzustände sind im Verfahrensfließbild der Vorbehandlung dargestellt. Die redundanten Ventile befinden sich je nach dem verwendeten Gasweg in entgegengesetztem Schaltzustand. In Betriebszustand 1 sind die Ventile (2a), (8b) und (6b) geöffnet, während die Ventile (2b), (8a) und (6a) geschlossen sind. Dadurch strömt das methanhaltige Rohgemisch über die Leitung (1a) zuerst in den Wärmetauscher (3a), danach in den Wärmetauscher (3b) und verlässt die Vorbehandlungseinrichtung über die Leitung (9). In diesem Betriebszustand durchströmt das Kühlmittel im Kühlkreislauf (10) ausschließlich den Wärmetauscher (3b). Der Wärmetauscher (3a) ist aus dem vorhergehenden Betriebzustand an seinen Außenwänden vereist und taut nun durch die Anströmung mit warmem methanhaltigem Rohgasgemisch ab, welches dadurch vorgekühlt wird. Im Kühlkreislauf (10) wird das darin zirkulierende Kühlmittel im Wärmetauscher (4) vom ausgetragenen Kohlendioxid in Leitung (5) abgekühlt und im Wärmetauscher (3b) zur Abkühlung des methanhaltigen Rohgemisches verwendet, wobei der im methanhaltigen Rohgemisch verbliebene Wasserdampf an den Wärmetauscherwänden gefriert. Ist der nicht mit Kühlmittel durchströmte Wärmetauscher vollständig abgetaut, wird in den Betriebzustand 2 umgeschaltet, in dem die Ventile (2a), (8b) und (6b) abgeschlossen und die Ventile (2b), (8a) und (6a) geöffnet werden. Dadurch kehrt sich die Strömungsrichtung des methanhaltigen Rohgasgemisches um, sodass der Wärmetauscher (3b) abgetaut wird. Das in diesem Vorbehandlungsprozess ausfallende Wasser fließt durch einen mit einem Siphon versehenen Abfluss (7) ab.

Der Vorbehandlungsprozess läuft drucklos, d.h. unter Atmosphärendruck, ab, wobei ein Volumenstrom des methanhaltigen Rohgemisches durch ein Gebläse lediglich in dem Maß erzeugt wird, dass der durch die Rohrreibung bedingte Druckverlust kompensiert wird.

Die erfindungsgemäße Vorrichtung und das erfindungsgemäße Verfahren erlauben eine energetisch effiziente Abtrennung von Methan aus methanhaltigen Gemischen und somit sowohl eine energetisch effiziente Herstellung von flüssigem Methan als auch eine energetisch effiziente Herstellung von gasförmigen Methan.

Auch wenn bevorzugte Ausführungsformen der erfindungsgemäßen Vorrichtung und des erfindungsgemäßen Verfahrens für methanhaltige Gasgemische, die im Wesentlichen aus Methan und Kohlendioxid bestehen (wie beispielsweise Biogas, Klärgas, Faulgas und Grubengas), beschrieben wurden, sind die erfindungsgemäße Vorrichtung und das erfindungsgemäßen Verfahren auch für methanhaltige Gasgemische geeignet, die zusätzlich zu oder anstelle von Kohlendioxid andere Gase enthalten.

### Bezugszeichenliste

- 1, 1a, 1b: Zuleitung Biogas
- 2a, 2b: Ventile der Zuleitung Biogas
- 3a, 3b: Wärmetauscher Biogas Vorbehandlung
- 4: Wärmetauscher Trockeneis
- 5: Austragsleitung Trockeneis
- 6a, 6b: Ventile Kühlkreislauf
- 7: Austragssiphon Wasser
- 8a, 8b: Ventile Biogas Schleife
- 9: Leitung Biogas vorbehandelt
- 10: Kühlkreislauf Vorbehandlung
- 11: Ventil Frisch-Methan
- 12: Biogaszufuhr Gastrennung
- 13: Ventil Methan Kühlkreislauf
- 14: Ventil LNG Kühlkreislauf
- 15: Zufuhrleitung LNG
- 16: Verflüssigungsaggregat
- 17: Zuleitung Tank
- 18: LNG-Tank
- 19: Kolben
- 20: Austragstrog
- 21: Plattenwärmetauscher
- 22: Auslass Frisch-Methan
- 23: Hydrauliksystem
- 24: Drei-Wege-Regler LNG
- 25: Oberfläche Wärmetauscher
- 26: Sammelbehälter Trockeneis
- 27: Schieber Trockeneisaustrag
- 28: Heizung Wärmetauscher
- 29: Methan Wärmetauscher
- 30: LNG Wärmetauscher
- 31: Trockeneisschicht
- 32: Auslass Methan Kühlkreislauf
- 33: Trockeneisbrocken
- 34: Trockeneispfropf
- 35: Zufuhrleitung Trockeneis
- 36: Vorlauf Kohlendioxid
- 37: Vorlauf Kühlmittel
- 38: Rücklauf Kühlmittel
- 39: Trockeneisschüttung
- 40: Kühlmittel
- 41: Gitter
- 42: Rost
- 43: Abtrennkammer
- 44: Drei-Wege-Regler
- 45: Kompressor
- 46: Zuleitung Gasnetz

## Patentansprüche

1. Vorrichtung zur Abtrennung von Methan aus methanhaltigen Gemischen, deren Komponenten eine höhere Siede-, Schmelz- oder Sublimationstemperatur als das flüssige Methan aufweisen, wobei die Vorrichtung mindestens einen Wärmetauscher mit einem Kühlmitteleinlass und einem Kühlmittelauslass, eine Abtrennkammer, welche den mindestens einen Wärmetauscher umschließt und einen Auslass für das gasförmige Methan aufweist, eine Verflüssigungseinheit, eine Leitung für das verflüssigte Methan und eine Leitung für das gasförmige Methan aufweist,
**dadurch gekennzeichnet, dass**
die Leitung für das verflüssigte Methan die Verflüssigungseinheit mit dem Kühlmitteleinlass des mindestens einen Wärmetauschers verbindet und die Leitung für das gasförmige Methan die Verflüssigungseinheit sowohl mit dem Kühlmittelauslass des mindestens einen Wärmetauschers als auch mit dem Auslass für das gasförmige Methan der Abtrennkammer verbindet.

2. Vorrichtung zur Abtrennung von Methan aus methanhaltigen Gemischen gemäß Anspruch 1, wobei der mindestens ein Wärmetauscher als Plattenwärmetauscher ausgeführt ist.

3. Vorrichtung zur Abtrennung von Methan aus methanhaltigen Gemischen gemäß Anspruch 1 oder 2, wobei die Platten des mindestens einen Wärmetauschers eine glatte Oberfläche aufweisen.

4. Vorrichtung zur Abtrennung von Methan aus methanhaltigen Gemischen gemäß einem der vorherigen Ansprüche, wobei der mindestens eine Wärmetauscher ein Heizelement aufweist.

5. Vorrichtung zur Abtrennung von Methan aus methanhaltigen Gemischen gemäß einem der vorherigen Ansprüche, wobei sowohl die Leitung für das flüssige Methan als auch die Leitung für das gasförmige Methan ein Ventil aufweist.

6. Vorrichtung zur Abtrennung von Methan aus methanhaltigen Gemischen gemäß einem der vorherigen Ansprüche, wobei die Vorrichtung ein Austragssystem für nicht methanhaltige Bestandteile aufweist.

7. Verfahren zur Abtrennung von Methan aus methanhaltigen Gemischen, deren Komponenten eine höhere Siede-, Schmelz- oder Sublimationstemperatur als das flüssige Methan aufweisen, wobei das methanhaltige Gemisch über einen Einlass in eine Abtrennkammer mit mindestens einem Wärmetauscher geleitet wird und das abgetrennte Methan über einen Auslass aus der Abtrennkammer geleitet wird,
**dadurch gekennzeichnet, dass**
das abgetrennte Methan aus dem Auslass der Abtrennkammer zusammen mit verdampften Methan aus dem mindestens einem Wärmetauscher zumindest teilweise in eine Verflüssigungseinheit geleitet und in der Verflüssigungseinheit verflüssigt wird und dass das verflüssigte Methan aus der Verflüssigungseinheit in den mindestens einen Wärmetauscher geleitet wird.

8. Verfahren zur Abtrennung von Methan aus methanhaltigen Gemischen gemäß Anspruch 7, wobei nur der Anteil des abgetrennten Methans in die Verflüssigungseinheit geleitet wird, der benötigt wird, um durch die Gastrennung verdampftes Methan aus dem mindestens einem Wärmetauscher durch flüssiges Methan als Kühlmittel zu ersetzen.

9. Verfahren zur Abtrennung von Methan aus methanhaltigen Gemischen gemäß einem der Ansprüche 7 oder 8, wobei das methanhaltige Gemische den mindestens einen Wärmetauscher überströmt, sodass die Komponenten des methanhaltigen Gemisches, die eine höhere Siede-, Schmelz- oder Sublimationstemperatur als das flüssige Methan aufweisen, an den Oberflächen des Wärmetauschers ausfrieren.

10. Verfahren zur Abtrennung von Methan aus methanhaltigen Gemischen gemäß Anspruch 9, wobei die an den Oberflächen des mindestens einen Wärmetauschers ausgefrorenen Komponenten des methanhaltigen Gemisches von der Oberfläche des mindestens einen Wärmetauschers abgelöst werden.

11. Verfahren zur Abtrennung von Methan aus methanhaltigen Gemischen gemäß Anspruch 10, wobei das Ablösen der an den Oberflächen des mindestens einen Wärmetauschers ausgefrorenen Komponenten des methanhaltigen Gemisches von der Oberfläche des mindestens einen Wärmetauschers dadurch erfolgt, dass die aus diesen Komponenten bestehende Schicht beim Abkühlen ihr Volumen verringert und dadurch Risse bekommt und/oder abplatzt.

12. Verfahren zur Abtrennung von Methan aus methanhaltigen Gemischen gemäß Anspruch 9 oder 10, wobei das Ablösen der an den Oberflächen des mindestens einen Wärmetauschers ausgefrorenen Komponenten des methanhaltigen Gemisches von der Oberfläche des mindestens einen Wärmetauschers durch Erwärmen des mindestens einen Wärmetauschers erfolgt.

13. Verfahren zur Abtrennung von Methan aus methanhaltigen Gemischen gemäß einem der Ansprüche 9-11. wobei das Ablösen der an den Oberflächen des mindestens einen Wärmetauschers ausgefrorenen Komponenten des methanhaltigen Gemisches von der Oberfläche des mindestens einen Wärmetauschers dadurch erfolgt, dass die Oberfläche des mindestens einen Wärmetauschers durch Erhöhen des Drucks im Inneren des mindestens einen Wärmetauschers nach außen gedrückt wird.

14. Verfahren zur Abtrennung von Methan aus methanhaltigen Gemischen gemäß Anspruch 13, wobei das Erhöhen des Drucks im Inneren des mindestens einen Wärmetauschers durch druckdichtes Abschließen des mindestens einen Wärmetauschers gegenüber dem mit dem mindestens einen Wärmetauscher verbundenen Leitungssystem und durch Erwärmen des Methans im Inneren des mindestens einen Wärmetauschers erfolgt.

15. Verfahren zur Abtrennung von Methan aus methanhaltigen Gemischen gemäß einem der Ansprüche 9 - 14, wobei als Stoffstrompumpe die Partialdruckdifferenz des methanhaltigen Rohgemisches am Einlass der Abtrennkammer zu dem abgetrennten Methan am Auslass der Abtrennkammer verwendet wird.

## Claims

1. A device for the separation of methane from methane-containing mixtures, the components of the mixtures having a higher boiling temperature, melting temperature or sublimation temperature than the liquid methane, wherein the device comprises at least one heat exchanger with a cooling agent inlet and a cooling agent outlet, a separation chamber enclosing the at least one heat exchanger and comprising an outlet for the gaseous methane, a liquefaction unit, a line for the liquefied methane and a line for the gaseous methane,
**characterized in that**
the line for the liquefied methane connects the liquefaction unit with the cooling agent inlet of the at least one heat exchanger and the line for the gaseous methane connects the liquefaction unit both with the cooling agent outlet of the at least one heat exchanger and with the outlet for the gaseous methane of the separation chamber.

2. The device for the separation of methane from methane-containing mixtures according to claim 1, wherein the at least one heat exchanger is realized as a plate-type heat exchanger.

3. The device for the separation of methane from methane-containing mixtures according to claim 1 or 2, wherein the plates of the at least one heat exchanger have a smooth surface.

4. The device for the separation of methane from methane-containing mixtures according to one of the preceding claims, wherein the at least one heat exchanger has a heating element.

5. The device for the separation of methane from methane-containing mixtures according to one of the preceding claims, wherein both the line for the liquid methane and the line for the gaseous methane have a valve.

6. The device for the separation of methane from methane-containing mixtures according to one of the preceding claims, wherein the device has a discharge system for non-methane containing components.

7. A method for the separation of methane from methane-containing mixtures, the components of the mixtures having a higher boiling temperature, melting temperature or sublimation temperature than the liquid methane, wherein the methane-containing mixture is led via an inlet into a separation chamber comprising at least one heat exchanger and the separated methane is led via an outlet out of the separation chamber,
**characterized in that**
the separated methane from the outlet of the separation chamber is led together with evaporated methane from the at least one heat exchanger at least partially into a liquefaction unit and liquefied in said liquefaction unit, and **in that** the liquefied methane is led out of the liquefaction unit into the at least one heat exchanger.

8. The method for the separation of methane from methane-containing mixtures according to claim 7, wherein the only portion of the separated methane that is led into the liquefaction unit is the portion required to replace the methane evaporated by the gas separation from the at least one heat exchanger by liquid methane as a cooling agent.

9. The method for the separation of methane from methane-containing mixtures according to one of claims 7 or 8, wherein the methane-containing mixture flows over the at least one heat exchanger, so that the components of the methane-containing mixture that have a higher boiling temperature, melting temperature or sublimation temperature than the liquid methane freeze out on the surfaces of the heat exchanger.

10. The method for the separation of methane from methane-containing mixtures according to claim 9, wherein the components of the methane-containing mixture that are frozen out on the surfaces of the at least one heat exchanger are separated from the surface of the at least one heat exchanger.

11. The method for the separation of methane from methane-containing mixtures according to claim 10, wherein the separation of the components of the methane-containing mixture that are frozen out on the surfaces of the at least one heat exchanger from the surface of the at least one heat exchanger takes place because the layer consisting of these components reduces its volume upon cooling and thus forms cracks and/or flakes off.

12. The method for the separation of methane from methane-containing mixtures according to claim 9 or 10, wherein the separation of the components of the methane-contain ing mixture that are frozen out on the surfaces of the at least one heat exchanger from the surface of the at least one heat exchanger takes place through the warming of the at least one heat exchanger.

13. The method for the separation of methane from methane-containing mixtures according to one of claims 9-11, wherein the separation of the components of the methane-containing mixture that are frozen out on the surfaces of the at least one heat exchanger from the surface of the at least one heat exchanger takes place because the surface of the at least one heat exchanger is pushed outwards by an increase of the pressure inside the at least one heat exchanger.

14. The method for the separation of methane from methane-containing mixtures according to claim 13, wherein the increase of the pressure inside the at least one heat exchanger takes place through the pressure-tight closure of the at least one heat exchanger against the system of lines connected with the at least one heat exchanger, and through the warming of the methane inside the at least one heat exchanger.

15. The method for the separation of methane from methane-containing mixtures according to one of claims 9 - 14, wherein the partial pressure difference between the methane-containing raw mixture at the inlet of the separation chamber and the separated methane at the outlet of the separation chamber is used as a material flow pump.

## Revendications

1. Dispositif pour la séparation de méthane de mélanges contenant du méthane, mélanges dont les composantes présentent une température d'ébullition, de fusion ou de sublimation supérieure à celle du méthane liquide, le dispositif présentant au moins un échangeur thermique avec un orifice d'admission de réfrigérant et une sortie de réfrigérant, une chambre de séparation qui englobe le ou les échangeurs thermiques et une sortie pour le méthane gazeux, une unité de liquéfaction, une conduite pour le méthane liquéfié et une conduite pour le méthane gazeux, **caractérisé en ce que**
la conduite de méthane liquéfié relie l'unité de liquéfaction avec l'orifice d'admission de réfrigérant du ou des échangeurs thermiques et la conduite de méthane gazeux relie l'unité de liquéfaction aussi bien à la sortie de réfrigérant du ou des échangeurs thermiques qu'à la sortie de méthane gazeux de la chambre de séparation.

2. Dispositif pour la séparation de méthane de mélanges contenant du méthane selon la revendication 1, le ou les échangeurs thermiques étant réalisé(s) comme échangeur(s) thermique(s) à plaques.

3. Dispositif pour la séparation de méthane de mélanges contenant du méthane selon la revendication 1 ou 2, les plaques du ou des échangeurs thermiques présentant une surface lisse.

4. Dispositif pour la séparation de méthane de mélanges contenant du méthane selon l'une quelconque des revendications précédentes, le ou les échangeurs thermiques présentant un élément chauffant.

5. Dispositif pour la séparation de méthane de mélanges contenant du méthane selon l'une quelconque des revendications précédentes, aussi bien la conduite de méthane liquide que la conduite de méthane gazeux présentant une soupape.

6. Dispositif pour la séparation de méthane de mélanges contenant du méthane selon l'une quelconque des revendications précédentes, le dispositif présentant un système de décharge de composants ne contenant pas du méthane.

7. Dispositif pour la séparation de méthane de mélanges contenant du méthane, mélanges dont les composantes présentent une température d'ébullition, de fusion ou de sublimation supérieure à celle du méthane liquide, le mélange contenant du méthane étant conduit via un orifice d'admission dans une chambre de séparation avec au moins un échangeur thermique et le méthane séparé étant conduit via une sortie hors de la chambre de séparation, **caractérisé en ce que**
le méthane séparé étant conduit au moins partiellement hors de la sortie de la chambre de séparation ensemble avec du méthane évaporé du ou des échangeurs thermiques dans une unité de liquéfaction et liquéfié dans l'unité de liquéfaction, et **en ce que** le méthane liquéfié étant conduit hors de l'unité de liquéfaction dans le ou les échangeurs thermiques.

8. Dispositif pour la séparation de méthane de mélanges contenant du méthane selon la revendication 7, seule la partie de méthane séparée qui est nécessaire pour remplacer du méthane évaporé par la séparation de gaz du ou des échangeurs thermiques par du méthane liquide comme réfrigérant étant conduite dans l'unité de liquéfaction.

9. Dispositif pour la séparation de méthane de mélanges contenant du méthane selon la revendication 7 ou 8, le mélange contenant du méthane inondant le ou les échangeurs thermiques de manière à ce que les composantes du mélange contenant du méthane, mélange présentant une température d'ébullition, de fusion ou de sublimation supérieure à celle du méthane liquide, se séparent par congélation aux surfaces de l'échangeur thermique.

10. Dispositif pour la séparation de méthane de mélanges contenant du méthane selon la revendication 9, les composantes du mélange contenant du méthane séparées par congélation aux surfaces du ou des échangeurs thermiques étant détachées.

11. Dispositif pour la séparation de méthane de mélanges contenant du méthane selon la revendication 10, le détachement des composantes du mélange contenant du méthane séparées par congélation aux surfaces du ou des échangeurs thermiques résultant de ce que la couche se composant de ces composantes réduit son volume lors du refroidissement et, de ce fait, subit des fissures et/ou s'effrite.

12. Dispositif pour la séparation de méthane de mélanges contenant du méthane selon la revendication 9 ou 10, le détachement des composantes du mélange contenant du méthane séparées par congélation aux surfaces du ou des échangeurs thermiques de la surface du ou des échangeurs thermiques résultant de l'échauffement du ou des échangeurs thermiques.

13. Procédé pour la séparation de méthane de mélanges contenant du méthane selon la revendication 9-11, le détachement des composantes du mélange contenant du méthane séparées par congélation aux surfaces du ou des échangeurs thermiques de la surface du ou des échangeurs thermiques résultant de ce que la surface du ou des échangeurs thermiques est pressée vers l'extérieur par augmentation de la pression à l'intérieur du ou des échangeurs thermiques.

14. Procédé pour la séparation de méthane de mélanges contenant du méthane selon la revendication 13, l'augmentation de la pression à l'intérieur du ou des échangeurs thermiques se faisant par fermeture étanche à la pression du ou des échangeurs thermiques par rapport au système de conduites relié au ou aux échangeurs thermiques et par échauffement du méthane à l'intérieur du ou des échangeurs thermiques.

15. Procédé pour la séparation de méthane de mélanges contenant du méthane selon la revendication 9-14, étant donné qu'on utilise comme pompe à courant de matière la différence de pression partielle du mélange brut contenant du méthane à l'orifice d'admission de la chambre de séparation par rapport au méthane séparé à la sortie de la chambre de séparation.
